Europäisches Patentamt

European Patent Office

Office européen des brevets

(19)

(11) Veröffentlichungsnummer: **0 004 355**

**B1**

(12) **EUROPÄISCHE PATENTSCHRIFT**

(21) Anmeldenummer: **79100807.1**

(22) Anmeldetag: **16.03.79**

(51) Int. Cl.³: **C 07 C 17/42**

(54) **Stabilisiertes Perchloräthylen**

(30) Priorität: **17.03.78 DE 2811779**
**13.12.78 DE 2853848**

(43) Veröffentlichungstag der Anmeldung:
**03.10.79 Patentblatt 79/20**

(45) Bekanntmachung des Hinweises auf die Patenterteilung:
**03.09.80 Patentblatt 80/18**

(84) Benannte Vertragsstaaten:
**BE CH DE FR GB IT SE**

(56) Entgegenhaltungen:
**CH - A - 593 350**
**DE - A - 2 449 667**
**FR - A - 2 119 014**
**US - A - 2 797 250**
**US - A - 3 133 885**

(73) Patentinhaber: **Wacker-Chemie GMBH**
**Prinzregentenstrasse 22**
**D - 8000 München 22 (DE)**

(72) Erfinder: **Dempf, Dominik, Dr. Dipl.-Chem.**
**Schifferlehnerstrasse 1**
**D - 8261 Mehring-Oed (DE)**
**Knabl, Rudolf**
**Robert-Koch-Strasse 55**
**D - 8263 Burghausen (DE)**
**Schmidhammer, Ludwig, Dr., Dipl.-Chem.**
**Pappelweg 5**
**D - 8261 Haiming**
**Markt 1 (DE)**
**Mack, Wilhelm, Dr., Dipl.-Chem.**
**Unghausen 24**
**D - 8263 Burghausen (DE)**

Courier Press, Leamington Spa, England.

## Stabilisiertes Perchloräthylen

Stabilisiertes Perchloräthylen ist bekannt. In der DE—OS 24 49 667 wird zur Stabilisierung die Zugabe von N-alkylmorpholin und Alkylphenol empfohlen. Aus der US-Patentschrift 3 133 885 ist es bekannt, zur Stabilisierung Amine und Oxirane einzusetzen. Stabilisiertes Perchloräthylen nach DE—OS 24 49 667 vermag bezüglich der Säureaufnahmefähigkeit den gestiegenen Anforderungen der Technik nicht mehr gerecht zu werden, solches, stabilisiert nach US—PS 3 133 885, erfüllt hinsichtlich der korrosiven Eigenschaften die technischen Anforderungen nur in unbefriedigender Weise. Schließlich ist auch stabilisiertes Perchloräthylen bekannt, das Phenole und Oxirane vereinigt, wobei gleichzeitig Anilin oder Pyrrole anwesend sein können. Zur Stabilisierung gegen oxidative Einflüsse müssen Anilin bzw. Pyrrol jedoch in vergleichsweise großen Mengen eingesetzt werden.

Um den gestiegenen Anforderungen gerecht zu werden, ist es Aufgabe der Erfindung, stabilisiertes Perchloräthylen anzugeben, das hinsichtlich Säureaufnahmefähigkeit und Korrosisonsverhalten sowie Stabilisierungswirkung bisherigem stabilisiertem Perchloräthylen überlegen ist.

Gegenstand der Erfindung ist stabilisiertes Perchloräthylen, das durch die Kombination von 0,001 bis 0,01 Gew.% wenigstens eines N-Alkylmorpholins, das gerade oder verzweigte Alkylgruppen mit 1 bis 5 Kohlenstoffatomen und ggf. Alkylsubstituenten mit bis zu 4 Kohlenstoffatomen am Ring enthält,

0,001 bis 0,01 Gew-%
       wenigens eines Alkylphenols, das wenigens eine gerade oder verzweigte Alkylgruppe mit 1 bis 18 Kohlenstoffatomen in o- und/oder p-Stellung enthält,

0,0005 bis 0,002 Gew.%
       Diisopropylamin und

0,05 bis 0,5 Gew.%
       Cyclohexenoxid gekennzeichnet ist.

Eine bevorzugte Ausführungsform des stabilisierten Perchloräthylens ist durch die Kombination von 0,004 bis 0,008 Gew.% N-Alkylmorpholin, 0,004 bis 0,008 Gew.% Alkylphenol, 0,0008 bis 0,0015 Gew.% Diisopropylamin und 0,1 bis 0,3 Gew.% Cyclohexenoxid gekennzeichnet.

Häufig ist es vorteilhaft, Epoxypropanol in Mengen zwischen 0,005 bis 0,1 Gew.% mit einzusetzen.

Unter den erfindungsgemäß genannten N-Alkylmorpholinen sind N-Methyl- oder N-Äthylmorpholin als besonders bevorzugt zu bezeichnen, unter den erfindungsgemäß genannten Alkylphenolen zeichnet sich in besonderer Weise das p.-tert.-Butylphenol aus.

Überraschenderweise war es möglich, durch Kombination bekannter stabilisierender Komponenten eine synergistisch gesteigerte Stabilisierung zu erreichen. Dies ist umso mehr überraschend, als durch die DE—OS 24 49 667 und DRP 573 105 bekannt war, daß die stabilisierende Wirkung von Alkylphenolen und N-Methylmorpholin durch die Zugabe von Diisopropylamin stark abgeschwächt wird. Durch die Mitverwendung von zusätzlichem Cyclohexenoxid wird dagegen die stabilisierende Wirkung, insbesondere gegen korrosiven Angriff, nicht abgeschwächt, sondern im Gegenteil wesentlich erhöht.

Diese Wirkung wird in zufriedenstellender Weise nur von Cyclohexenoxid erfüllt.

Durch die Zugabe von Epoxypropanol wird ein zusätzlicher Schutz gegen unerwünschte Umlagerung von Cyclohexenoxid erreicht.

Stabilisiertes Perchloräthylen soll bereits bei einem geringen Gehalt stabilisierender Komponenten eine langanhaltende stabilisierende Wirkung auf Perchloräthylen gegen oxidative Zersetzung beim Kontakt mit Metallen, Legierungen, Metallsalzen, Schneidölen und Einwirkung von Licht und thermischer Belastung aufweisen und darüberhinaus dem Perchloräthylen eine langanhaltende gesteigerte Säureaufnahmefähigkeit verleihen. Insbesondere soll die Stabilität gegenüber Metallen wie Eisen, Kupfer, Zink und Aluminium gut sein und Korrosion an diesen Metallen verhindert werden.

Die Stabilisatorkomponenten sind im Perchloräthylen in gelöster Form enthalten.

Unter N-Alkylmorpholin wird erfindungsgemäß Morpholin mit verzweigten oder geraden Alkylketten mit 1 bis 5 Kohlenstoffatomen verstanden, weiterhin kann der Ring in 2-, 3-, 5- oder 6- Stellung mit Alkylketten mit bis zu 4 Kohlenstoffatomen substituiert sein. Vorzugsweise wird N-Methyl- bzw. N-Äthylmorpholin eingesetzt.

Unter Alkylphenolen werden o- und/oder p-Alkylphenole mit 1 bis 18 Kohlenstoffatomen mit gerader oder verzweigter Alkylkette verstanden. Vorzugsweise werden solche Alkylphenole eingesetzt, die 2 bis 8 Kohlenstoffatome in der Alkylkette aufweisen, insbesondere solche Alkylphenole, die eine verzweigte Alkylkette mit 3 bis 5 Kohlenstoffatomen in p-Stellung aufweisen, auch im Gemisch, verwendet.

Besonders gute Stabilisierung erhält man mit einem Gemisch aus N-Methylmorpholin, p.-tert.-Butylphenol, Diisopropylamin und Cyclohexenoxid. Die Stabilisatorwirksamkeit gegen oxidative Einflüsse wird durch Untersuchungen entsprechend den Bedingungen des MIL-Tests 7003 ermittelt. Dazu werden in einem Testkolben 200 ml Perchloräthylen von unten durch eine mattierte 150 Watt Glühlampe beheizt und belichtet. Das dabei siedende Perchloräthylen kommt mit einem Stahlstreifen von 12,7 mm x 50,8 mm x 1,59 mm, der mit einem VA-Draht in einem auf den Kolben aufgesetzten Rückflußkühler aufgehängt ist, in Berührung. Ein zweiter Stahlstreifen von 6,35 mm × 19,5 mm × 1,59 mm wird auf den Kolbenboden in die siedende Flüssigkeit gelegt. Durch ein Glasrohr mit 3 mm Durchmesser, das 6,35 mm über dem Kolbenboden endet, wird ein mit Wasser gesättigter Sauerstoffstrom von 10 bis 12 Blasen/Minute in das Perchloräthylen eingeleitet. Vom abgekühlten Kolbeninhalt wird eine Probe entnommen wird und im wäßrigen Auszug durch Titration mit 0,1 N-Natronlauge gegen Phenolphthalein die Acidität bestimmt. Proben, deren Säuregehalt nach dem 48-stündigen Test 0,02 Gew.% Salzsäure übersteigen, sind unwirksam stabilisiert. Das Maß für den Stabilisierungseffekt ist die Zeit bis zur Erreichung der Aciditätsgrenze von 0,02 Gew.% Salzsäure im Perchloräthylen, ausgedrückt in Stunden. Die Säureaufnahmefähigkeit von stabilisiertem Perchloräthylen bestimmt man wie folgt:

25 cm³ Hydrochlorierungslösung (hergestellt aus 1 cm³ konzentrierter Salzsäure und 250 cm³ Methanol) werden mit 25 cm³ destilliertem Wasser in einen Erlenmeyerkolben gefüllt und mit 0,1 N-Natronlauge gegen Phenolphthalein bis zur bleibenden Rosafärbung titriert (Verbrauch A in ml). Dann werden 25 cm³ des Hydrochlorierungsreagenzes und 15 cm³ Probe in einen Kolben pipettiert und am Rückfluß 5 Minuten erwärmt. Nach Abkühlen werden 25 cm³ destilliertes Wasser zugegeben und mit 0,1 N-Natronlauge titriert (Verbrauch B in ml). Die Säureaufnahmefähigkeit berechnet sich zu

$$\frac{(A - B) \times 0,4}{V \times D} = \text{Gew.\% Natronlauge}$$

V = Volumen der Probe in cm³
D = Dichte der Probe in g/cm³

Die Stabilisatorwirksamkeit gegenüber Korrosion wird durch einfache Tests demonstriert, wobei Proben von Metallstreifen aus Eisen, Kupfer, Aluminium, Zink, die teilweise in die Flüssigphase eintauchen, mit Perchloräthylen 168 Stunden am Rückfluß gekocht werden. Das Ausmaß der Verfärbung bzw. Teerbildung an der Metalloberfläche nach Testende ist ein Maß für die korrosiven Eigenschaften des stabilisierten Perchloräthylens.

Die folgenden Beispiele, die in der Tabelle 1 zusammengefaßt sind, zeigen in deutlicher Weise die Überlegenheit der erfindungsgemäßen Stabilisierung.

TABELLE I

| Nr. | ppm Stabilisator | Stunden bis zur Erreichung der Aciditäts-grenze | SAF % NaOH nach 72 h Rückfluβ*) | Korrosions-verhalten gegenüber Fe, Cu, Zn, Al |
|---|---|---|---|---|
| 1 | 15 Diisopropylamin | | | starke Korrosion Cu, Zn |
| | 50 N-Methylmorpholin | 100 | 0,01 | |
| | 30 p.-tert.-Butyl-phenol | | | |
| 2 | 50 N-Methylmorpholin | | | leichte Korrosion Fe, Cu, Zn |
| | 30 p.-tert.-Butyl-phenol | 145 | 0,01 | |
| 3 | 50 N-Methylmorpholin | | | Korrosion Fe, Zn |
| | 30 p.-tert.-Butyl-phenol | 180 | 0,06 | |
| | 2 500 Cyclohexenoxid | | | |
| 4 | 15 Diisopropylamin | | | leichte Korrosion gegenüber Fe, Cu, Al |
| | 30 p.-tert.-Butyl-phenol | 70 | 0,06 | |
| | 2 500 Cyclohexenoxid | | | |
| 5 | 15 Diisopropylamin | | | keine Korrosion gegenüber Fe, Cu, Al, Zn |
| | 50 N-Methylmorpholin | | | |
| | 30 p.-tert.-Butyl-phenol | 390 | 0,07 | |
| | 2 500 Cyclohexenoxid | | | |
| 6 | 15 Diisopropylamin | | | leichte Korrosion an Fe, Zn, Cu |
| | 50 N-Methylmorpholin | | | |
| | 30 p.-tert.-Butyl-phenol | 175 | 0,04 | |
| | 2 500 Propylenoxid | | | |
| 7 | 15 Diisopropylamin | | | Korrosion an Fe, Cu, Zn |
| | 50 N-Methylmorpholin | | | |
| | 30 p.-tert.-Butyl-phenol | 180 | 0,05 | |
| | 2 500 Butylenoxid | | | |

TABELLE I (cont.)

| Nr | ppm Stabilisator | Stunden bis zur Errei- chung der Aciditats- grenze | SAF % NaOH nach 72 h Ruckfluβ*) | Korrosions- verhalten gegenuber Fe, Cu, Zn, Al |
|---|---|---|---|---|
| 8 | 15 Diisopropylamin | | | leichte Korrosion an Fe, Zn |
| | 30 N-Methylmorpholin | | | |
| | 30 p.-tert.-Butyl- phenol | 190 | 0,06 | |
| | 2 500 Epichlorhydrin | | | |
| 9 | 15 Anilin | | | starke Korrosion an Cu, Fe, Zn |
| | 50 N-Methylmorpholin | | | |
| | 30 p.-tert.-Butyl phenol | 170 | 0,06 | |
| | 2 500 Cyclohexenoxid | | | |

*) und anschließendem, zehnmaligem Destillieren

SAF = Säureaufnahmefähigkeit

## Beispiel 10

Zusatz von Epoxipropanol: Perchloräthylen wurde mit 50 ppm N-Methylmorpholin, 15 ppm Diisopropylamin, 50 ppm p.-tert-Butylphenol, 2500 ppm Cyclohexenaoxid und 200 ppm Epoxipropanol stabilisiert. Dann wurden in einem ersten Versuch 200 ppm und in einem zweiten Versuch 400 ppm Zinkchlorid zugefügt und anschließend 72 Stunden zum Sieden erhitzt. Nach dieser Zeit war das Cyclohexenoxid unverändert vorhanden. Im Gaschromatogramm wurden 2460 ppm Cyclohexenoxid wiedergefunden, Cyclopentylaldehyd war auch nicht in Spuren nachweisbar, das Gemisch zeigte keinen Fremdgeruch.

## Patentansprüche

1. Stabilisiertes Perchloräthylen, gekennzeichnet durch die Kombination von 0,001 bis 0,01 Gew.% wenigstens eines N-Alkylmorpholins, das gerade oder verzweigte Alkylgruppen mit 1 bis 5 Kohlenstoffatomen und ggf. Alkylsubstituenten mit bis zu 4 Kohlenstoffatomen am Ring enthält, 0,001 bis 0,01 Gew.%
wenigstens eines Alkylphenols das wenigstens eine gerade oder verzweigte Alkylgruppe mit 1 bis 18 Kohlenstoffatomen in o- und/oder p-Stellung enthält, 0,0005 bis 0,002 Gew.%
Diisopropylamin
0,05 bis 0,5 Gew.%
Cyclohexenoxid und gegebenenfalls
0,005 bis 0,1 Gew.%
Epoxypropanol.

2. Stabilisiertes Perchloräthylen nach Anspruch 1, gekennzeichnet durch die Kombination von 0,004 bis 0,008 Gew.% N-Alkylmorpholin, 0,004 bis 0,008 Gew.%
Alkylphenol
0,0008 bis 0,0015 Gew.%
Diisopropylamin,
0,1 bis 0,3 Gew.%
Cyclohexenoxid und gegebenenfalls
0,005 bis 0,1 Gew.%
Epoxipropanol.

# 0 004 355

3. Stabilisiertes Perchloräthylen nach Anspruch 1 dadurch gekennzeichnet, daß es als N-Alkylmorpholin N-Methyl- oder N-Äthylmorpholin und als Alkylphenol p.-tert.-Butylphenol enthält.

## Revendications

1. Perchloréthylène stabilisé, caractérisé par l'association de 0,001 à 0,01% en poids d'au moins une N-alkyl-morpholine qui porte des radicaux alkyles, linéaires ou ramifiés, contenant de 1 à 5 atomes de carbone et, éventuellement, sur le noyau, des substituants alkyliques contenant au plus 4 atomes de carbone,
de 0,001 à 0,01% en poids
d'au moins un alkyl-phenol qui porte, en ortho et/ou en para, au moins un radical alkyle, linéaire ou ramiflé, contenant de 1 à 18 atomes de carbone,
de 0,0005 à 0,002% en poids
de diisopropylamine,
de 0,05 à 0,5% en poids
d'oxyde de cyclohexène et, éventuellement,
de 0,005 à 0,1% en poids
d'époxypropanol.
2. Perchloréthylène stabilisé selon la revendication 1, caractérisé par l'association de 0,004 à 0,008% en poids d'une N-alkyl-morpholine,
de 0,004 à 0,008% en poids
d'un alkyl-phénol
de 0,008 à 0,0015% en poids
de diisopropylamine,
de 0,1 à 0,3% en poids
d'oxyde de cyclohexène et, éventuellement,
de 0,005 à 0,1% en poids
d'époxypropanol.
3. Perchloréthylène stabilisé selon la revendication 1, caractérisé par le fait qu'il contient comme N-alkyl-morpholine la N-méthyl-morpholine ou la N-éthyl-morpholine, et comme alkyl-phénol le p-tert-butyl-phénol.

## Claims

1. Stabilized perchloroethylene characterized by the combination of 0.001 to 0.1% by weight of at least one N-alkylmorpholine containing straight- or branched-chain alkyl groups having from 1 to 5 carbon atoms and optionally alkyl substitutents having up to 4 carbon atoms in the ring,
0.001 to 0.01% by weight
of at least one alkylphenol containing at least one straight- or branched-chain alkyl group having from 1 to 18 carbon atoms in o- or p-positions.
0.0005 to 0.002% by weight
of diisopropylamine,
0.05 to 0.5% by weight
of cyclohexene oxide, and optionally
0.005 to 0.1% by weight
of epoxypropanol.
2. Stabilized perchloroethylene according to claim 1, characterized by the combination of 0.004 to 0.008% by weight of N-alkyl-morpholine,
0.004 to 0.008% by weight
of alkylphenol,
0.0008 to 0.0015% by weight
of diisopropylamine,
0.1 to 0.3% by weight
of cyclohexene oxide, and optionally
0.005 to 0.1% by weight
of epoxypropanol.
3. Stabilized perchloroethylene according to claim 1, characterized in that it contains as N-alkyl-morpholine N-methyl- or N-ethyl-morpholine and as alkylphenol p.-tert.-butylphenol.